# EUROPÄISCHE PATENTSCHRIFT

(11) **EP 2 496 276 B1**
(45) Veröffentlichungstag und Bekanntmachung des Hinweises auf die Patenterteilung: **28.08.2013**
(21) Anmeldenummer: 10773537.5
(22) Anmeldetag: 01.11.2010
(51) Int. Cl.: A61M 1/00, G01F 23/26

(54) **DRAINAGEPUMPEINHEIT**
DRAINAGE PUMP UNIT
ENSEMBLE POMPE DE DRAINAGE

(30) Priorität: 05.11.2009 CH 17062009
(43) Veröffentlichungstag der Anmeldung: 12.09.2012
(73) Patentinhaber: Medela Holding AG, 6340 Baar (CH)
(72) Erfinder: GIEZENDANNER, Charles, CH-6443 Morschach (CH); GUGL, Christoph, CH-8032 Zürich (CH)
(74) Vertreter: Clerc, Natalia
(86) Internationale Anmeldenummer: PCT/CH2010/000273
(87) Internationale Veröffentlichungsnummer: WO 2011/054118

(56) Entgegenhaltungen:
- EP-A1- 0 853 950
- US-A- 4 092 860
- US-A- 4 898 593

## Beschreibung

### TECHNISCHES GEBIET

Die vorliegende Erfindung betrifft eine Drainagepumpeinheit gemäss Oberbegriff des Patentanspruchs 1.

### STAND DER TECHNIK

Zum Absaugen von Körperflüssigkeiten bzw. -fluiden im medizinischen Bereich werden Drainagepumpsysteme eingesetzt. Sie werden beispielsweise bei bzw. nach chirurgischen Eingriffen, in der Wunddrainage, der Thoraxdrainage oder beim Absaugen von Körperfetten eingesetzt. Diese Drainagepumpsysteme weisen üblicherweise eine Vakuumpumpe, eine oder mehrere Fluidsammelbehälter und eine Drainageschlauchverbindung zwischen Patient und Fluidsammelbehälter auf. Der Fluidsammelbehälter kann am Gehäuse der Drainagepumpe lösbar befestigt sein oder er ist mit der Pumpe über einen externen Vakuumschlauch verbunden.

Indem mittels der Saug- bzw. Vakuumpumpe ein Unterdruck im Fluidsammelbehälter erzeugt wird, lässt sich das Fluid oder das Sekret von einer Kavität des Patienten über den Drainage- oder Sekretschlauch in den Sammelbehälter saugen und dort sammeln. Am pumpenseitigen Ausgang des Sammelbehälters angeordnete Filter schützen die Saugpumpe vor einer allfälligen Verunreinigung durch das abgesaugte Fluid. Derartige Drainagepumpsysteme sind beispielsweise in WO 2007/128156 und in WO 2008/141471 offenbart. Die dort beschriebenen Drainagepumpsystem sind tragbar ausgebildet.

Zur Überwachung des Drainageverlaufs und des Füllstandes des Fluidsammelbehälters sind kapazitive Füllstandsensoren geeignet. Derartige Füllstandsensoren sind im Stand der Technik bekannt. So offenbaren JP 2161322 und US 4 099 167 kapazitive Füllstandsensoren zur Bestimmung eines Füllstandes eines Flüssigkeitsbehälters, welche auf der Aussenseite des Flüssigkeitsbehälters angebracht sind.

US 4 092 860 offenbart einen auf der Aussenseite eines Stutzens aufgebrachten kapazitiven Füllstandsensor. Dieser Sensor ist gegen aussen mit einer Teflonschicht geschützt.

DE 196 45 970 offenbart einen bandförmigen kapazitiven Füllstandsensor, welcher im Innern eines Behälters angeordnet ist.

Bei Drainagesystemen zum Absaugen von Körperflüssigkeiten befinden sich die Füllstandsensoren üblicherweise am oder im Fluidsammelbehälter. So offenbart WO 2008/119993 einen Fluidsammelbehälter mit mehreren auf der Aussenseite des Behälters angebrachten Füllstandsensoren. EP 0 853 950 hingegen offenbart eine Drainagepumpeinheit mit einer Saugeinheit und einem Fluidsammelbehälter, bei welcher ein kapazitiver Füllstandsensor an der Saugeinheit angeordnet ist.

Fluidsammelbehälter können aus hygienischen Gründen nur eine begrenzte Zeit eingesetzt werden und sollten deshalb möglichst kostengünstig sein. Werden die Füllstandsensoren am Fluidsammelbehälter angebracht, erhöhten sich jedoch deren Kosten. Füllstandsensoren, welche an der Drainagepumpeinheit angebracht sind, weisen hingegen im Vergleich zu behälterseitigen Füllstandsensoren eine grössere Ungenauigkeit auf, insbesondere bei kleinen Füllmengen.

### DARSTELLUNG DER ERFINDUNG

Es ist deshalb eine Aufgabe der Erfindung, eine Drainagepumpeinheit zu schaffen, welche die oben genannten Nachteile behebt.

Diese Aufgabe löst eine Drainagepumpeinheit mit den Merkmalen des Patentanspruchs 1.

Die erfindungsgemässe Drainagepumpeinheit zum Absaugen von Körperfluiden mittels einer Saugpumpe weist ein Saugpumpengehäuse und eine darin angeordnete Saugpumpe auf. Am Saugpumpengehäuse ist ein Fluidsammelbehälter lösbar befestigbar. Am Saugpumpengehäuse ist ferner ein flachbandförmiger kapazitiver Füllstandsensor angeordnet zur Detektion eines Füllstandes im Fluidsammelbehälter. Erfindungsgemäss weist die Drainagepumpeinheit ein elastisch ausgebildetes Kopplungselement mit einer ersten Oberfläche und einer der ersten Oberfläche gegenüberliegenden zweiten Oberfläche auf, wobei das Kopplungselement im befestigten Zustand des Fluidsammelbehälters mit der ersten Oberfläche am Füllstandsensor und mit der zweiten Oberfläche an einer Aussenwand des Fluidsammelbehälters anliegt.

Dank dieses Kopplungselements lässt sich ein allfälliger Luftspalt zwischen Fluidsammelbehälter und der sensitiven Fläche des Füllstandsensors vermeiden. Die Messgenauigkeit ist erhöht und es können bereits Füllstände bereits zwischen 0 und 200 ml oder zwischen 0 und 300 ml in ml-Schritten gemessen werden. Diese Anordnung ist insbesondere zur kontinuierlichen Füllstandmessung und zur Bestimmung der Füllrate geeignet.

Diese Anordnung lässt sich auch mit einem oder mehreren Neigungssensoren, welche vorzugsweise ebenfalls am Saugpumpengehäuse angeordnet sind, kombinieren. Durch Verwendung von mehr als einem Füllstandsensor und geeignete Anordnung derselben lässt sich die Neigung des Sammelbehälters auch bestimmen, so dass keine gesonderten Neigungssensoren eingesetzt werden müssen.

Die Elastizität des Kopplungselements lässt sich einerseits durch geeignete Materialwahl und andererseits durch geeignete Formgebung erzielen. Vorzugsweise wird ein Material verwendet, dessen kapazitive Eigenschaften denjenigen des Fluidsammelbehälters gleichen.

Das Kopplungselement kann am Fluidsammelbehälter angeordnet sein. Vorzugsweise ist es jedoch am Saugpumpengehäuse angeordnet.

In beiden Fällen kann der Fluidsammelbehälter ohne Sensoren ausgebildet sein. Dadurch lässt er sich kostengünstig herstellen und als Einwegprodukt ausbilden. Er kann somit nach erstmaligem Gebrauch entsorgt werden.

Dank der Elastizität des Kopplungselements bleibt die Funktionsfähigkeit des Sensors und des Messsystems auch nach mehrfachem Wechseln des Fluidsammelbehälters erhalten.

Ist das Kopplungselement am Saugpumpengehäuse angeordnet und überdeckt somit die Sensorfläche des Füllstandsensors, so ist diese Sensorfläche vor äusseren Einflüssen geschützt und das Gehäuse ist einfacher zu reinigen als bei freiliegenden Sensorflächen.

In einer bevorzugten Ausführungsform ist der Füllstandsensor in einer äusseren Seitenwand des Saugpumpengehäuses angeordnet, wobei das Kopplungselement dieser Seitenwand vorsteht. Es wird bei Befestigung des Fluidsammelbehälters am Gehäuse elastisch eingedrückt und gewährleistet so einen optimalen und einheitlichen dielektrischen Übergang von der Sensorfläche des Füllstandsensors zur äusseren Behälterwand.

Das Kopplungselement kann auf dem Füllstandsensor, insbesondere auf seiner sensitiven Oberfläche, befestigt, insbesondere auf ihr aufgeschweisst, aufgeschmolzen oder mit ihr vergossen sein. Vorzugsweise ist der Füllstandsensor jedoch im Kopplungselement eingebettet. Dadurch kann das Kopplungselement gleichzeitig als Montage- und Befestigungsmittel zur Befestigung des Füllstandstandsensors im Saugpumpengehäuse dienen. Das Kopplungselement kann im Falle des eingebetteten Füllstandsensors auch gleichzeitig oder alternativ als Schutzhülle für den Füllstandsensor dienen.

In einer bevorzugten Ausführungsform weist diese Schutzhülle eine Basis auf, deren zwei gegenüberliegende Oberflächen die oben genannte erste Oberfläche und die zweite Oberfläche bilden. Diese Basis ist von einer mindestens teilweise umlaufenden Seitenwand umgeben, welche einen Hohlraum bildet, so dass der Füllstandsensor in diesem Hohlraum angeordnet werden kann.

Zur Befestigung in einer Öffnung des Saugpumpengehäuses ist an dem der Basis gegenüberliegenden Ende der Seitenwand ein mindestens teilweise umlaufender nach aussen vorstehender erster Flansch angeformt und an der Basis ist ein nach aussen vorstehender zweiter Flansch angeformt. Diese zwei Flansche liegen beidseits der Öffnung des Gehäuses an.

Vorzugsweise ist der Füllstandsensor im Bereich unterhalb eines zur Saugpumpe führenden Vakuumanschlusses des Saugpumpengehäuses angeordnet. Alternativ oder zusätzlich kann er auch schräg beabstandet zu einem Sekretanschluss des Saugpumpengehäuses, welcher eine Verbindung mit einem patientenseitigen Sekretschlauch ermöglicht, angeordnet sein. In all diesen Fällen wird eine Verschmutzung des Sensors durch allfällige aus dem Sekretanschluss austretende Fluidtropfen weitgehend vermieden.

Weitere Ausführungsformen sind in den abhängigen Ansprüchen angegeben.

### KURZE BESCHREIBUNG DER ZEICHNUNGEN

Eine bevorzugte Ausführungsform der Erfindung wird im Folgenden anhand der Zeichnungen beschrieben, die lediglich zur Erläuterung dienen und nicht einschränkend auszulegen sind. In den Zeichnungen zeigen:
- Figur 1: eine perspektivische Darstellung einer Drainagepumpeinheit mit einem Fluidsammelbehälter gemäss der Erfindung;
- Figur 2: die Drainagenpumpeinheit gemäss Figur 1 ohne Fluidsammelbehälter;
- Figur 3: eine erste Seitenansicht eines erfindungsgemässen Kopplungselements mit darin eingebettetem Füllstandsensor;
- Figur 4: das Kopplungselement gemäss Figur 3 in einer Ansicht von unten;
- Figur 5: einen Schnitt durch das Kopplungselement gemäss Figur 3 entlang C-C;
- Figur 6: das Kopplungselement gemäss Figur 3 in einer zweiten Seitenansicht und
- Figur 7: eine perspektivische Darstellung des Kopplungselements gemäss Figur 3.

### BESCHREIBUNG BEVORZUGTER AUSFÜHRUNGSFORMEN

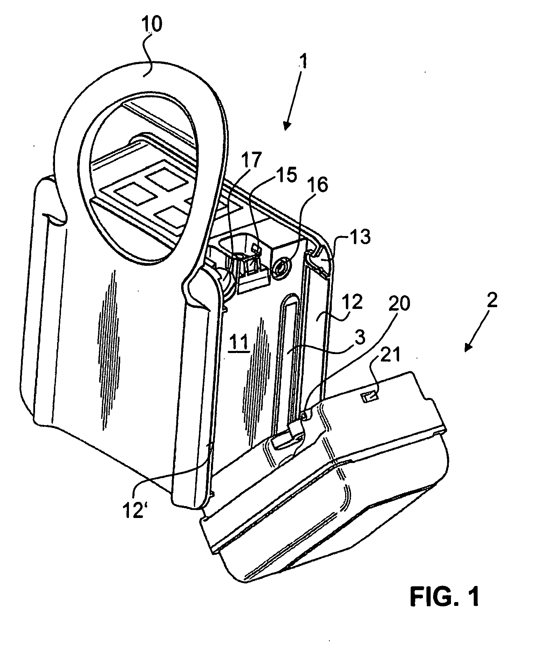

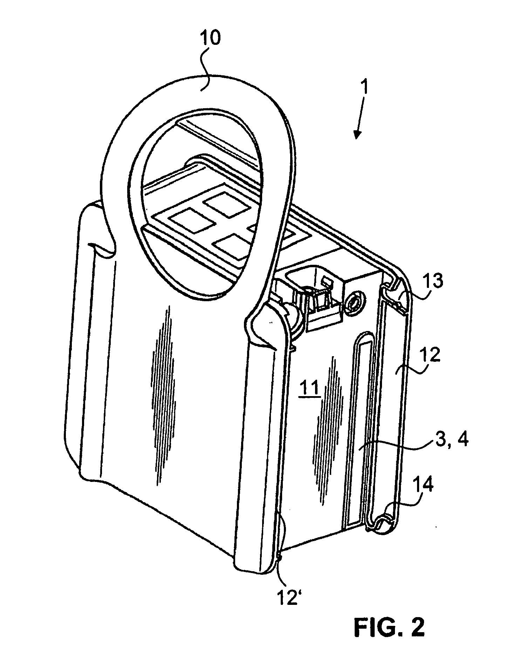

In den Figuren 1 und 2 ist eine Drainagepumpeinheit dargestellt, wie sie im Grundaufbau beispielsweise aus WO 2007/128156 und WO 2008/141471 bekannt ist. Sie weist ein Saugpumpengehäuse 1 mit einer Vorderwand 12, einer Rückwand 12' und zwei Seitenwänden 11 auf. Das Gehäuse 1 kann tragbar ausgebildet sein, beispielsweise mittels eines Traggriffs 10. Vorzugsweise weist das Gehäuse 1 alternativ oder zusätzlich zum Traggriff 10 eine Standfläche oder Standfüsse auf, so dass das Gehäuse in der abgebildeten Lage auf einem Tisch oder einer anderen geeigneten Standfläche aufgestellt werden kann. Nachfolgend angegebene Richtungsangaben sind in dieser stehenden Position des Gehäuses zu verstehen.

Im Gehäuse 1 sind eine nicht sichtbare Saug- oder Vakuumpumpe und Elektronik zum Betrieb der Pumpe sowie zur Auswertung von Messdaten einer allfälligen mit Sensoren versehenen Serviceleitung angeordnet. Die Saugpumpe ist über ein am Gehäuse angeordnetes Bedienfeld oder Bedienungstasten und/oder knöpfe bedienbar.

Das Saugpumpengehäuse 1 ist in diesem Beispiel quaderförmig ausgebildet, wobei mindestens eine seitliche äussere Gehäusewand 11 im Wesentlichen plan ausgebildet ist. Das Saugpumpengehäuse 1 kann jedoch auch eine andere Form aufweisen.

An der planen seitlichen Gehäusewand 11 stehen die Vorderwand 12 und die Rückwand 12' seitlich vor und bilden zusammen mit der seitlichen Gehäusewand 11 eine Aufnahme für einen Fluidsammelbehälter 2.

Dieser Fluidsammelbehälter 2 ist vorzugsweise im Wesentlichen quaderförmig ausgebildet. Das Saugpumpengehäuse 1 und der Fluidsammelbehälter 2 sind vorzugsweise aus Kunststoff gefertigt.

Der Fluidsammelbehälter 2 ist lösbar am Saugpumpengehäuse 1 befestigt. Vorzugsweise wird er dabei eingeschwenkt und rastet in dieser Position ein. Hierzu weisen der vorstehende Teil der vorderen Wand 12 und der Rückwand 12' obere und untere Kulissenführungen 13, 14 auf, in welche obere und untere Einrastbolzen 20 des Fluidsammelbehälters 2 eingreifen. In Figur 1 ist nur ein oberer Einrastbolzen 20 sichtbar. Der Fluidsammelbehälter 2 weist zwei einander gegenüberliegende obere und zwei einander gegenüberliegende untere Einrastbolzen 20 auf, welche alle derselben äusseren Seitenwand des Behälters 2 vorstehen. Mit dieser Seitenwand liegt der Fluidsammelbehälter 2 an der Seitenwand 11 des Saugpumpengehäuses 1 an. Die Seitenwand des Fluidsammelbehälters 2 ist vorzugsweise ebenfalls im Wesentlichen plan ausgebildet.

Der Sammelbehälter 2 weist eine Ausnehmung 21 auf, in welche ein am Gehäuse 1 angeordneter Einrasthaken 15 einrastet und so den Sammelbehälter 2 in seiner Lage am Gehäuse 1 lösbar fixiert. Anstelle von Kulissenführungen, Einrastbolzen und Einrasthaken kann die Befestigung des Sammelbehälters 2 und seine Fixierung auch mit anderen Mitteln erfolgen.

In der Seitenwand 11 des Gehäuses 1 ist ein gehäuseseitiger Vakuumanschluss 16 vorhanden. Vorzugsweise ist er in einem oberen Bereich der Seitenwand 11 angeordnet. Der Vakuumanschluss 16 weist in diesem Beispiel die Form eines Stutzens auf, welcher in eine hier nicht dargestellte entsprechende Öffnung des Fluidsammelbehälters 2 eingreift. Das freie Ende des Stutzens 16 fluchtet dabei mit der Seitenwand 11 des Gehäuses oder ist sogar relativ zu ihr in Richtung Gehäuse 1 zurückversetzt. Vorzugsweise steht der Stutzen 16 somit nicht vor. Damit die durch den Stutzen 16 gebildete Vakuumverbindung zwischen Gehäuse 1 und Behälter 2 dicht ist, weist der Stutzen 16 des Gehäuses 1 und oder die Öffnung des Behälters 2 einen hier nicht dargestellten Dichtring auf. Damit im Behälter 2 gesammeltes abgesaugtes Sekret nicht in das Gehäuse 1 gelangen kann, ist der Vakuumanschluss 16 und/oder die zugehörige Öffnung des Behälters 2 vorzugsweise mit einem oder mehreren Filtern versehen.

Im oberen Bereich der Seitenwand 11, an der dem Vakuurnanschluss 16 gegenüberliegenden Seite, ist ein gehäuseseitiger Sekretanschluss bzw. eine Ausnehmung 17 zur Anbringung eines derartigen Anschlusses vorhanden. Hier lässt sich ein pumpenseitiges Anschlussteil eines patientenseitigen Drainageschlauchs einstecken, wie dies in WO 2008/141471 offenbart ist. Dieser Sekretanschluss 17 wird wie der Vakuumanschluss 16 dichtend mit einer entsprechenden Öffnung des Fluidsammelbehälters 2 verbunden, wenn der Fluidsammelbehälter 2 am Gehäuse 1 befestigt, hier eingeschwenkt, wird. Es ist jedoch auch möglich, die Verbindung zwischen Behälter 2 und patientenseitigem Drainageschlauch auf andere Art und Weise zu erstellen, beispielsweise kann der Schlauch auch direkt bzw. über geeignete Kopplungsteile im Behälter 2 einsteckbar sein.

Über den Vakuumanschluss 16 wird mittels der Saugpumpe ein Unterdruck im Behälter 2 erzeugt. Dank diesem Unterdruck wird ein Fluid oder Sekret aus der Kavität des Patienten durch den Drainageschlauch in den Behälter 2 gesaugt und dort gesammelt.

Die Vorrichtung umfasst mindestens einen flachbandförmigen kapazitiven Füllstandsensor 4. Derartige Füllstandsensoren sind aus dem Stand der Technik bekannt. Sie sind plan und flach ausgebildet. Ihre Länge ist wesentlich grösser als ihre Breite und ihre Dicke und ihre Breite ist zudem üblicherweise wesentlich grösser als ihre Dicke.

Eine hier nicht dargestellte Auswerteeinheit ist mit dem kapazitiven Füllstandsensor 4 verbunden. Diese Einheit befindet sich vorzugsweise im Gehäuse 1. Der kapazitive Füllstandsensor 4 ermöglicht vorzugsweise eine kontinuierliche Messung des Füllstandes und ermöglicht somit auch die Bestimmung einer Füllrate. Es ist jedoch auch möglich, in Intervallen oder nach Massgabe des Benutzers zu messen.

Der Füllstandsensor 4 ist in der Seitenwand 11 des Gehäuses 1 angeordnet, wobei seine sensitive Fläche vorzugsweise zum Behälter 2 hin gerichtet ist. Diese sensitive Fläche und somit der Sensor 4 erstrecken sich in ihrer Längrichtung vorzugsweise in vertikaler Richtung von unten nach oben. Sie können jedoch auch schräg angeordnet sein. Des Weiteren können mehr als ein, insbesondere zwei oder drei Füllstandsensoren 4 vorhanden sein. Diese können an verschiedenen Stellen und/oder auf verschiedenen Höhen in vertikaler Richtung verlaufen. Ein Füllstandsensor 4 kann sich jedoch auch in seiner Längsrichtung in vertikaler Richtung erstrecken und ein anderer Füllstandsensor kann schräg dazu angeordnet sein. Auf diese Weise lässt sich bei der Füllstandsmessung auch die Neigung des Gehäuses und des Behälters bestimmen, so dass die Messdaten betreffend Füllstand entsprechend korrigiert werden können. Es lassen sich jedoch auch andere Arten von Neigungssensoren gemeinsam mit dem Füllstandsensor verwenden.

Zwischen Sensor 4 und Behälter 2 ist ein elastisch ausgebildetes Kopplungselement 3 vorhanden. Dieses Kopplungselement 3 weist eine Basis 30 mit zwei einander gegenüberliegenden, planparallelen Oberflächen 300 auf. Der Sensor 4 liegt im befestigten Zustand des Behälters 2 vorzugsweise mit seiner sensitiven Fläche an einer dieser Oberflächen an. Eine äussere Wand des Behälters 2 liegt in diesem Zustand an der zweiten dieser Oberflächen an. Vorzugsweise liegt diese zweite Oberfläche 300 mindestens mit einem Flächeninhalt am Behälter 2 an, welcher dem Flächeninhalt der sensitiven Fläche entspricht. D.h. sie stellt eine Verbindung der gesamten sensitiven Fläche zur Behälterwand her.

Das Kopplungselement 3 ist vorzugsweise aus Silikon, PVC oder Kautschuk gefertigt. Andere Materialien mit geeigneten dielektrischen Eigenschaften sind jedoch auch einsetzbar. Vorzugsweise weist das Kopplungselement ähnliche kapazitive Eigenschaften auf wie das Material des Behälters 2.

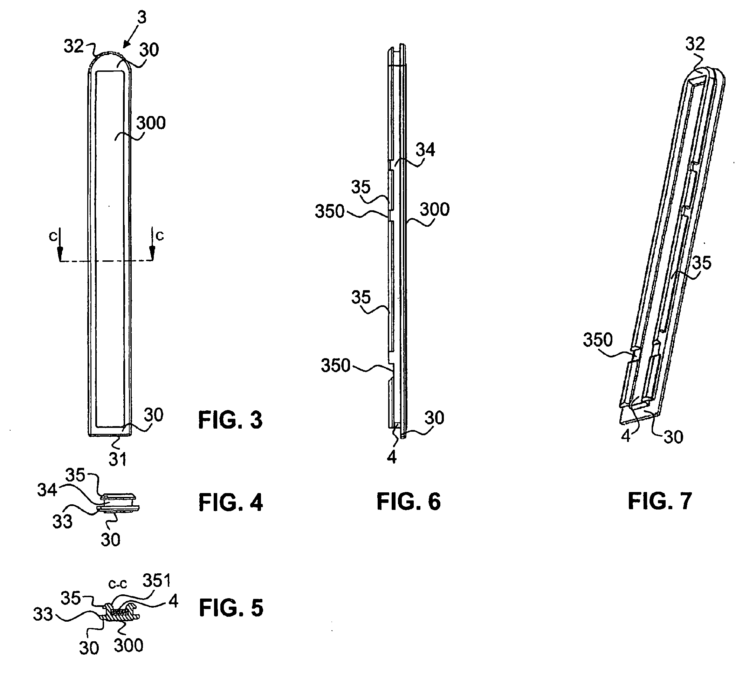

In einer einfachen Ausführungsform ist der Sensor 4 im Gehäuse 1 gehalten und seine nach aussen gerichtete sensitive Fläche ist von diesem Kopplungselement 3 ganzflächig überdeckt. Vorzugsweise bildet das Kopplungselement 3 jedoch eine Sensorhülle, wie sie in den Figuren 3 bis 7 gut erkennbar ist und wie sie in den Figuren 1 und 2 in das Gehäuse 1 eingesetzt ist.

Die Sensorhülle 3 ist vorzugsweise einstückig ausgebildet. Ihre Länge entspricht mindestens der Länge des Sensors 4. Sie weist die Basis 30 mit den zwei planparallelen Oberflächen auf. Auf der äusseren, im montierten Zustand dem Gehäuse 1 abgewandten Oberfläche ist eine plane Kontaktfläche 300 vorhanden. Der Flächeninhalt der Kontaktfläche 300 entspricht mindestens dem Flächeninhalt der sensitiven Fläche, wobei die sensitive Fläche vollständig von dieser Kontaktfläche 300 überdeckt ist. Vorzugsweise ist diese plane Kontaktfläche 300 der sie umgebenden restlichen äusseren Oberfläche leicht vorstehend ausgebildet. Dies ist in Figur 6 gut erkennbar.

Auf der inneren, im montierten Zustand dem Gehäuse 1 zugewandten Seite der Basis 30 ist eine mindestens teilweise umlaufende Seitenwand 34 angeformt. Die Seitenwand 34 erstreckt sich über die zwei langen Seiten der Basis 30 und einer kurzen Seite. Die andere kurze Seite wird offen gelassen. Die Sensorhülle weist somit ein offenes Ende 31 und ein geschlossenes, hier zudem abgerundetes Ende 32 auf.

Der freie Rand dieser Seitenwand 34 geht in einen nach aussen ragenden ersten Flansch 35 über, welcher vorzugsweise einen nach innen ragenden Anteil 351 aufweist. Die Seitenwand 34 ist zurückversetzt auf der Basis 30 angeformt, so dass auch diese einen nach aussen ragenden zweiten Flansch 33 bildet, welcher beabstandet aber vorzugsweise parallel zum ersten Flansch 35 verläuft. Der zweite Flansch 33 ist vorzugsweise ohne Unterbruch umlaufend ausgebildet. Der erste Flansch 35 kann Unterbrechungen 350 aufweisen.

Wie in den Figuren 5, 6 und 7 erkennbar ist, lässt sich der flachbandförmige kapazitive Sensor 4 in diese Sensorhülle 3 einschieben und wird in dem durch die umlaufende Seitenwand 34 und die Basis 30 gebildeten Hohlraum gehalten. Dabei fixiert der nach innen ragende Anteil 351 des ersten Flansches 35 den Sensor 4 in seiner Lage. Die sensitive Fläche des Sensors 4 liegt auf der planen Innenseite der Basis 30 an und hat ihr Gegenstück an der zu ihr vorzugsweise planparallelen äusseren Kontaktfläche 300 der Sensorhülle 3.

Die Sensorhülle 3 lässt sich nun in die Öffnung der Gehäuseseitenwand 11 einrasten, wobei der erste Flansch 35 auf die Innenseite des Gehäuses 1 zu liegen kommt und der zweite Flansch 33 auf der Aussenseite der Gehäusewand 11 anliegt. Vorzugsweise entspricht deshalb die Höhe der Sensorhüllen-Seitenwand 34 annähernd der Dicke der Gehäuseseitenwand 11.

Wird nun der Fluidsammelbehälter 2 am Gehäuse 1 befestigt, so liegt der Behälter 2 mit seiner planen Aussenfläche an der gesamten Kontaktfläche 300 an. Vorzugsweise verformt der Behälter 2 dabei die Kontaktfläche 300 und drückt sie in Richtung Gehäuse 1, so dass eine optimale Kontaktierung entgegen einer Federkraft erreicht wird. Die ganze Anordnung ist deshalb vorzugsweise so zu bemessen, dass die Kontaktfläche 300 eingedrückt werden kann.

Die hier beschriebene Drainagepumpeinheit ist lediglich ein Ausführungsbeispiel zur Anwendung der erfinderischen Lehre. Das Pumpengehäuse sowie der Drainagebehälter können beispielsweise eine andere Form aufweisen. Ferner können die Vakuum- und Sekretanschlüsse anders ausgestaltet und an anderen Stellen angeordnet sein. Das Kopplungselement kann anders, insbesondere nicht als Sensorhülle, ausbildet sein. Weitere Änderungen sind möglich.

Die erfindungsgemässe Drainagepumpeinheit ermöglicht dank des elastisch ausgebildeten Kopplungselements eine Füllstandsmessung bereits bei kleinen Füllmengen.

### BEZUGSZEICHENLISTE

| | | | |
|---|---|---|---|
| 1 | Saugpumpengehäuse | 21 | Ausnehmung |
| 10 | Traggriff | | |
| 11 | seitliche Gehäusewand | 3 | Kopplungselement |
| 12 | Vorderwand | 30 | Basis |
| 12' | Rückwand | 300 | Kontaktfläche |
| 13 | obere Kulissenführung | 31 | offenes Ende |
| 14 | untere Kulissenführung | 32 | geschlossenes Ende |
| 15 | Verriegelungsmechanismus | 33 | zweiter Flansch |
| 16 | gehäuseseitiger | 34 | Seitenwand |
| | Vakuumanschluss | 35 | erster Flansch |
| 17 | Ausnehmung/gehäuseseitiger | 351 | nach innen ragender Anteil |
| | Sekretanschluss | 350 | Unterbrechungen |
| | | | |
| 2 | Fluidsammelbehälter | 4 | Füllstandsensor |
| 20 | Einrastbolzen | | |

## Patentansprüche

1. Drainagepumpeinheit zum Absaugen von Körperfluiden mittels einer Saugpumpe, wobei die Drainagepumpeinheit ein Saugpumpengehäuse (1) und eine darin angeordnete Saugpumpe aufweist, wobei am Saugpumpengehäuse (1) ein Fluidsammelbehälter (2) lösbar befestigbar ist und wobei am Saugpumpengehäuse (1) ein flachbandförmiger kapazitiver Füllstandsensor (4) angeordnet ist zur Detektion eines Füllstandes im Fluidsammelbehälter (2), **dadurch gekennzeichnet, dass** die Drainagepumpeinheit ferner ein elastisch ausgebildetes Kopplungselement (3) mit einer ersten Oberfläche und einer der ersten Oberfläche gegenüberliegenden zweiten Oberfläche aufweist, wobei das Kopplungselement (3) im befestigten Zustand des Fluidsammelbehälters (2) mit der ersten Oberfläche am Füllstandsensor (4) und mit der zweiten Oberfläche an einer Aussenwand des Fluidsammelbehälters (2) anliegt.

2. Drainagepumpeinheit nach Anspruch 1, wobei das Kopplungselement (3) am Saugpumpengehäuse (1) angeordnet ist.

3. Drainagepumpeinheit nach Anspruch 2, wobei der Füllstandsensor (4) in einer äusseren Seitenwand (11) des Saugpumpengehäuses (1) angeordnet ist und das Kopplungselement (3) dieser Seitenwand (11) vorsteht.

4. Drainagepumpeinheit nach einem der Ansprüche 2 oder 3, wobei der Füllstandsensor (4) im Kopplungselement (3) eingebettet ist.

5. Drainagepumpeinheit nach einem der Ansprüche 2 bis 4, wobei das Kopplungselement (3) eine Basis (30) aufweist, welche die erste und zweite Oberfläche bildet, und wobei diese Basis (30) von einer mindestens teilweise umlaufenden Seitenwand (34) umgeben ist, welche einen Hohlraum bildet und wobei der Füllstandsensor (4) in diesem Hohlraum angeordnet ist.

6. Drainagepumpeinheit nach Anspruch 5, wobei an dem der Basis (30) gegenüberliegenden Ende der Seitenwand (34) des Kopplungselements (3) ein mindestens teilweise umlaufender nach aussen vorstehender erster Flansch (35) angeformt ist und an der Basis (30) ein nach aussen vorstehender zweiter Flansch (33) angeformt ist, wobei die zwei Flansche (34, 35) zur Befestigung des Kopplungselements (3) in einer Öffnung der äusseren Seitenwand (11) des Saugpumpengehäuses (1) dienen.

7. Drainagepumpeinheit nach einem der Ansprüche 1 bis 6, wobei die erste und die zweite Oberfläche plan ausgebildet sind und sich mindestens über die gesamte Sensorfläche des Füllstandsensors (4) erstrecken.

8. Drainagepumpeinheit nach einem der Ansprüche 1 bis 7, wobei das Kopplungselement (3) einstückig ausgebildet ist.

9. Drainagepumpeinheit nach einem der Ansprüche 1 bis 8, wobei der Füllstandsensor (4) eine Längsrichtung aufweist und wobei er in dieser Längsrichtung vertikal verlaufend am Saugpumpengehäuse (1) angeordnet ist.

10. Drainagepumpeinheit nach einem der Ansprüche 1 bis 9, wobei der Füllstandsensor (4) im Bereich unterhalb eines zur Saugpumpe führenden Vakuumanschlusses (16) des Saugpumpengehäuses (1) angeordnet ist.

11. Drainagepumpeinheit nach einem der Ansprüche 1 bis 10, wobei der Füllstandsensor (4) schräg beabstandet zu einem Sekretanschluss (17) des Saugpumpengehäuses (1) zur Verbindung mit einem patientenseitigen Sekretschlauch angeordnet ist.

12. Drainagepumpeinheit nach einem der Ansprüche 1 bis 11, wobei das Saugpumpengehäuse (1) eine äussere Seitenwand (11) aufweist und wobei der Füllstandsensor (4) sowie mindestens einer der folgenden Anschlüsse, Sekretanschluss (17) und Vakuumanschluss (16), in dieser Seitenwand (11) angeordnet sind.

13. Drainagepumpeinheit nach einem der Ansprüche 1 bis 12, wobei das Kopplungselement (4) aus Silikon oder PVC oder Kautschuk besteht.

14. Drainagepumpeinheit nach Anspruch 1, wobei die Drainagepumpeinheit den Fluidsammelbehälter (2) umfasst und wobei das Kopplungselement (4) am Fluidsammelbehälter (2) angeordnet ist.

## Claims

1. Drainage pump unit for aspirating body fluids by means of a suction pump, the drainage pump unit comprising a suction pump housing (1) and a suction pump arranged in the latter, a fluid collection container (2) being able to be secured releasably on the suction pump housing (1), and a flat band-shaped capacitive filling level sensor (4) being arranged on the suction pump housing (1) for the purpose of detecting a filling level in the fluid collection container (2), **characterized in that** the drainage pump unit also comprises an elastically configured coupling element (3) with a first surface and a second surface lying opposite the first surface, and the coupling element (3), in the secured state of the fluid collection container (2), bears with the first surface on the filling level sensor (4) and with the second surface on an outer wall of the fluid collection container (2).

2. Drainage pump unit according to Claim 1, in which the coupling element (3) is arranged on the suction pump housing (1).

3. Drainage pump unit according to Claim 2, in which the filling level sensor (4) is arranged in an outer side wall (11) of the suction pump housing (1), and the coupling element (3) protrudes from this side wall (11).

4. Drainage pump unit according to one of Claims 2 and 3, in which the filling level sensor (4) is embedded in the coupling element (3).

5. Drainage pump unit according to one of Claims 2 to 4, in which the coupling element (3) has a base (30) that forms the first surface and second surface, and in which this base (30) is surrounded by an at least partially encircling side wall (34) that forms a hollow space, and the filling level sensor (4) is arranged in this hollow space.

6. Drainage pump unit according to Claim 5, in which an at least partially encircling and outwardly protruding first flange (35) is formed integrally on that end of the side wall (34) of the coupling element (3) opposite the base (30), and an outwardly protruding second flange (33) is formed integrally on the base (30), the two flanges (34, 35) serving to secure the coupling element (3) in an opening of the outer side wall (11) of the suction pump housing (1).

7. Drainage pump unit according to one of Claims 1 to 6, in which the first surface and second surface are plane and extend at least across the entire sensor face of the filling level sensor (4).

8. Drainage pump unit according to one of Claims 1 to 7, in which the coupling element (3) is designed in one piece.

9. Drainage pump unit according to one of Claims 1 to 8, in which the filling level sensor (4) has a longitudinal direction and is arranged extending vertically on the suction pump housing (1) in this longitudinal direction.

10. Drainage pump unit according to one of Claims 1 to 9, in which the filling level sensor (4) is arranged in the area below a vacuum attachment part (16) of the suction pump housing (1) leading to the suction pump.

11. Drainage pump unit according to one of Claims 1 to 10, in which the filling level sensor (4) is arranged obliquely at a distance from a secretion attachment part (17) of the suction pump housing (1) for connection to a patient-side secretion tube.

12. Drainage pump unit according to one of Claims 1 to 11, in which the suction pump housing (1) has an outer side wall (11), and in which the filling level sensor (4) and at least one of the following attachment parts, secretion attachment part (17) and vacuum attachment part (16), are arranged in this side wall (11).

13. Drainage pump unit according to one of Claims 1 to 12, in which the coupling element (3) is made of silicone or PVC or rubber.

14. Drainage pump unit according to Claim 1, in which the drainage pump unit comprises the fluid collection container (2), and in which the coupling element (3) is arranged on the fluid collection container (2).

## Revendications

1. Unité de pompe de drainage pour aspirer des fluides corporels au moyen d'une pompe d'aspiration, l'unité de pompe de drainage présentant un boîtier de pompe d'aspiration (1) et une pompe d'aspiration disposée dans celui-ci, un réservoir de collecte de fluide (2) pouvant être fixé de manière détachable au boîtier de pompe d'aspiration (1) et un capteur de niveau de remplissage capacitif en forme de bande plate (4) étant disposé sur le boîtier de pompe d'aspiration (1) pour détecter un niveau de remplissage dans le réservoir de collecte de fluide (2), **caractérisée en ce que** l'unité de pompe de drainage présente en outre un élément d'accouplement (3) réalisé sous forme élastique avec une première surface et une deuxième surface opposée à la première surface, l'élément d'accouplement (3), dans l'état fixé du réservoir de collecte de fluide (2), s'appliquant avec la première surface contre le capteur de niveau de remplissage (4) et avec la deuxième surface contre une paroi extérieure du réservoir de collecte de fluide (2).

2. Unité de pompe de drainage selon la revendication 1, dans laquelle l'élément d'accouplement (3) est disposé sur le boîtier de pompe d'aspiration (1).

3. Unité de pompe de drainage selon la revendication 2, dans laquelle le capteur de niveau de remplissage (4) est disposé dans une paroi latérale extérieure (11) du boîtier de pompe d'aspiration (1) et l'élément d'accouplement (3) fait saillie hors de cette paroi latérale (11).

4. Unité de pompe de drainage selon l'une quelconque des revendications 2 ou 3, dans lequel le capteur de niveau de remplissage (4) est encastré dans l'élément d'accouplement (3).

5. Unité de pompe de drainage selon l'une quelconque des revendications 2 à 4, dans lequel l'élément d'accouplement (3) présente une base (30), qui forme la première et la deuxième surface, cette base (30) étant entourée par une paroi latérale (34) au moins partiellement périphérique, qui forme un espace creux, le capteur de niveau de remplissage (4) étant disposé dans cet espace creux.

6. Unité de pompe de drainage selon la revendication 5, dans laquelle à l'extrémité de la paroi latérale (34) de l'élément d'accouplement (3) opposée à la base (30) est façonnée une première bride (35) au moins en partie périphérique faisant saillie vers l'extérieur et une deuxième bride (33) faisant saillie vers l'extérieur est façonnée au niveau de la base (30), les deux brides (34, 35) servant à la fixation de l'élément d'accouplement (3) dans une ouverture de la paroi latérale extérieure (11) du boîtier de pompe d'aspiration (1).

7. Unité de pompe de drainage selon l'une quelconque des revendications 1 à 6, dans laquelle la première et la deuxième surface sont réalisées sous forme plane et s'étendent au moins sur toute la surface de capteur du capteur de niveau de remplissage (4).

8. Unité de pompe de drainage selon l'une quelconque des revendications 1 à 7, dans laquelle l'élément d'accouplement (3) est réalisé d'une seule pièce.

9. Unité de pompe de drainage selon l'une quelconque des revendications 1 à 8, dans laquelle le capteur de niveau de remplissage (4) présente une direction longitudinale et il est disposé dans cette direction longitudinale en s'étendant verticalement sur le boîtier de pompe d'aspiration (1).

10. Unité de pompe de drainage selon l'une quelconque des revendications 1 à 9, dans lequel le capteur de niveau de remplissage (4) est disposé dans la région en dessous d'un raccord à vide (16) du boîtier de pompe d'aspiration (1) conduisant à la pompe d'aspiration.

11. Unité de pompe de drainage selon l'une quelconque des revendications 1 à 10, dans laquelle le capteur de niveau de remplissage (4) est disposé obliquement à distance d'un raccord de sécrétions (17) du boîtier de pompe d'aspiration (1) en vue de la connexion à un tuyau de sécrétion provenant du patient.

12. Unité de pompe de drainage selon l'une quelconque des revendications 1 à 11, dans laquelle le boîtier de pompe d'aspiration (1) présente une paroi latérale extérieure (11) et dans laquelle le capteur de niveau de remplissage (4) ainsi qu'au moins l'un des raccords suivants, le raccord de sécrétions (17) et le raccord à vide (16), sont disposés dans cette paroi latérale (11).

13. Unité de pompe de drainage selon l'une quelconque des revendications 1 à 12, dans laquelle l'élément d'accouplement (4) se compose de silicone ou de PVC ou de caoutchouc.

14. Unité de pompe de drainage selon la revendication 1, dans laquelle l'unité de pompe de drainage comprend le réservoir de collecte de fluide (2) et dans laquelle l'élément d'accouplement (4) est disposé sur le réservoir de collecte de fluide (2).
